# EUROPEAN PATENT APPLICATION

(11) **EP 1 889 919 A1**
(43) Date of publication of application: **20.02.2008**
(21) Application number: 06746662.3
(22) Date of filing: 19.05.2006
(51) Int. Cl.: C12Q 1/37, G01N 33/543, G01N 33/573

(54) **METHOD OF ANALYZING ENZYME**

(30) Priority: 20.05.2005 JP 2005148793
(71) Applicant: Mitsubishi Kagaku Iatron, Inc., Tokyo 162-0812 (JP)
(72) Inventor: OGURA, Minoru c/o MITSUBISHI KAGAKU IATRON, INC.,, Tokyo 1620812 (JP); ONO, Tomoko c/o MITSUBISHI KAGAKU IATRON, INC.,, Tokyo 1620812 (JP)
(74) Representative: Vossius & Partner
(86) International application number: PCT/JP2006/310055
(87) International publication number: WO 2006/123789

(57) **Abstract**

A method of analyzing an enzyme, comprising (1) bringing a sample suspected of containing an enzyme to be analyzed into contact with an enzyme substrate which is immobilized an insoluble carrier and can be cleaved by the enzyme, in a liquid, (2) separating the insoluble carrier from the liquid, and (3) analyzing a substrate or a fragment thereof which remains on the insoluble carrier and/or a fragment of the substrate which is released from the insoluble carrier into the liquid; and a kit for analyzing an enzyme, comprising at least an enzyme substrate which is immobilized on an insoluble carrier and can be cleaved by the enzyme, are disclosed.

SEQUENCE LISTING

<110> Mitsubishi Kagaku Iatron, Inc.

<120> Enzyme assay

<130> N3245 EP

<150> JP 2005-148793
<151> 2005-05-20

<160> 1

<170> PatentIn version 3.1

<210> 1
<211> 4
<212> PRT
<213> Artificial Sequence

<220>
<221> Source
<223> /note="Description of Artificial Sequence: a synthetic substrate"

<400> 1

Ile Glu Gly Arg
1

## Description

### TECHNICAL FIELD

The present invention relates to a method of analyzing an enzyme contained in a sample. More particularly, the present invention relates to a method of analyzing an enzyme, for example, in a measurement of an enzyme activity, by reacting a sample with a substrate for the enzyme to be measured which is immobilized on an insoluble carrier (i.e., an immobilized substrate), and detecting an amount of a fragment generated by the cleavage of the substrate with the enzyme.

### BACKGROUND ART

In the field of clinical diagnostic tests, biological components, which may be used as an index of diagnosis of diseases, in a large number of samples should be measured rapidly and accurately and a rapid and accurate feedback of the results should be given to a treating room. This enables an effective selection of an appropriate drug or treatment, an effective follow-up, and an effective prediction of recurrence.

Biological components used as an index of diagnosis of diseases, so-called diagnostic markers, are manifold, for example, proteins, nucleic acids, enzymes, polysaccharides, lipid, and the like. For an early diagnosis of diseases, a method capable of measuring a very little trace amount of a biological component becomes important.

Most biological components are catalysts and inhibitory activities thereagainst, particularly, enzymes and inhibitors thereagainst, in chemical reactions. Enzyme reactions in vivo are regulated and adjusted by activators and inhibitors, and as a result, functions are controlled. For example, the coagulation and fibrinolysis system in vivo is involved in many enzymes and inhibitors, and the measurement of these factors becomes clinically important.

As an assay for enzymes, an assay using a synthetic substrate or an immunoassay is used in accordance with an enzyme to be analyzed. In the assay using a synthetic substrate, an activating factor can be detected or quantitatively analyzed by measuring a chromogenic substnace or a fluorescent substance released by the acting of an enzyme to be analyzed.

For example, thrombin, an endoprotease involved in blood coagulation, is an important enzyme which cleaves fibrinogen to generate fibrin. As its synthetic substrate, for example, Bz-Phe-Val-Arg-pNA (non-patent reference 1), Tos-Gly-Pro-Arg-pNA (patent reference 1), or H-D-Phe-Pip-Arg-pNA (patent reference 2) is known. When such a substrate is reacted with thrombin, para-nitroaniline (pNA) is released from the substrate to develop a yellow color. A activity of thrombin may be determined by colorimetric measurement at 405 nm, and the activity of thrombin in coagulation and fibrinolysis can be indicated with an enzyme activity.

Further, the activity of Tissue Factor (hereinafter referred to as TF) may be determined using a chromogenic synthetic substrate [non-patent reference 2 and non-patent reference 3].

The activities of activating factors other than thrombin and TF in the coagulation and fibrinolysis system, such as plasmin, antithrombin III (AT III), von Willebrand Factor cleaving protease (ADAMTS13), Factor X, Factor XI, Factor XII, protein C, plasma kallikrein, tissue plasminogen activator, or urokinase, are measured using synthetic substrates.

As a method of analyzing a trace amount of biological components, an enzyme assay does not require a pretreatment for separating a subject to be analyzed from a sample (such a pretreatment is essential for common assays), because the subject can be specifically selected from the sample mixture containing complicated components by an enzyme-substrate reaction. Further, in an enzyme assay, the subject can be quantitatively determined with a high sensitivity and a high accuracy. In enzyme assays, an enzyme assay characterized by a fluorescent or enzymatic labeling may be carried out at a low cost, without hazardous wastes, and having the same sensitivity and accuracy, in comparison with a radiolabeled enzyme assay using a radioisotope, and thus, is widely used.

As described above, with respect to many activating factors such as enzymes, various analytic systems have been developed by utilizing the substrate specificities. Further, a substrate having a higher specificity has been developed, and a more accurate measurement (improvement of substrate specificity) is now possible by the development of synthetic substrates. However, these synthetic substrates basically develop color or fluorescence after the release of a chromogenic or fluorescent substance from the synthetic substrate. However such a signal is not always generated under any condition, and it is necessary to bind a specific substance to the specific site of a chromogenic or fluorescent substance. Namely, the development of novel synthetic substrates is not easy.

Further, when an enzymatic reaction is carried out in a liquid phase and the development is detected in the liquid phase, the assay is susceptible of turbidity or a color of a sample. Since fluorescence assay is also susceptible of a contaminant which emits fluorescence, the measurement values become inaccurate.

[patent reference 1] Japanese Unexamined Patent Publication (Kokai) No. 52-3492
[patent reference 2] Japanese Unexamined Patent Publication (Kokai) No. 52-24590
[non-patent reference 1] Thrombosis Research, Ireland, 1972, vol. 1, p. 267-278
[non-patent reference 2] Clinical Chemistry, the U.S.A., 1989, vol. 35, p. 1897
[non-patent reference 3] Rinsho Byori (The Japanese Journal of Clinical Pathology), 1995, vol. 43, p. 137

### DISCLOSURE OF THE INVENTION

### PROBLEMS TO BE SOLVED BY THE INVENTION

An object of the present invention is to provide a method with an excellent operability and a high sensitivity, in a method of analyzing an enzyme by using a substrate for the enzyme.

### MEANS FOR SOLVING THE PROBLEMS

The object can be solved by the present invention, a method of analyzing an enzyme, characterized by comprising the steps of:
(1) bringing a sample suspected of containing an enzyme to be analyzed into contact with an enzyme substrate which is immobilized on an insoluble carrier and can be cleaved by the enzyme, in a liquid (hereinafter referred to as contacting step),
(2) separating the insoluble carrier from the liquid (hereinafter referred to as separating step), and
(3) analyzing a substrate or a fragment thereof which remains on the insoluble carrier and/or a fragment of the substrate which is released from the insoluble carrier into the liquid (hereinafter referred to as analyzing step).

In a preferred embodiment of the present invention, the enzyme substrate is labeled with a labeling substance at a side of a substrate fragment released from the insoluble carrier by a cleavage with the enzyme to be analyzed.
In another preferred embodiment of the present invention, the step (3) is carried out by using a labeled partner which is labeled with a labeling substance and specifically binds to a neoantigen which is newly generated by the cleavage of the enzyme substrate with the enzyme to be analyzed.
In still another preferred embodiment of the present invention, the labeling partner is a labeling antibody.
The term "neoantigen" as used herein means a cleaved site which is newly generated, by cleaving an enzyme substrate with an enzyme of interest, on a substrate fragment bound to an insoluble carrier or a released substrate fragment. As the cleaved site of an enzyme substrate, there may be mentioned, for example, a peptide sequence, or a changed tertiary structure of an enzyme substrate by the cleavage.

In still another preferred embodiment of the present invention, the labeling substance is a fluorescent substance, a luminescent substance, a chromogenic substance, or an enzyme.
In still another preferred embodiment of the present invention, the insoluble carrier is a particle.
In still another preferred embodiment of the present invention, the particle is a latex particle or a magnetic particle.

The present invention relates to a kit for analyzing an enzyme, characterized by comprising an enzyme substrate which is immobilized on an insoluble carrier and can be cleaved by the enzyme, wherein the enzyme substrate is labeled with a labeling substance at a side of a substrate fragment released from the insoluble carrier by a cleavage with the enzyme.
The present invention relates to a kit for analyzing an enzyme, characterized by comprising an enzyme substrate which is immobilized on an insoluble carrier and can be cleaved by the enzyme, and a labeled partner which is labeled with a labeling substance and specifically binds to a neoantigen which is newly generated by a cleavage of the enzyme substrate with the enzyme.

The term "analysis" as used herein includes a detection of a presence or absence of a substance to be analyzed, and a measurement to quantitatively or semi-quantitatively determine an amount or activity of a substance to be analyzed.

### EFFECTS OF THE INVENTION

According to the present invention, in a measurement of an amount of an enzyme, a method of conveniently and quickly detecting the enzyme with a high sensitivity can be provided.

### BRIEF DESCRIPTION OF THE DRAWINGS

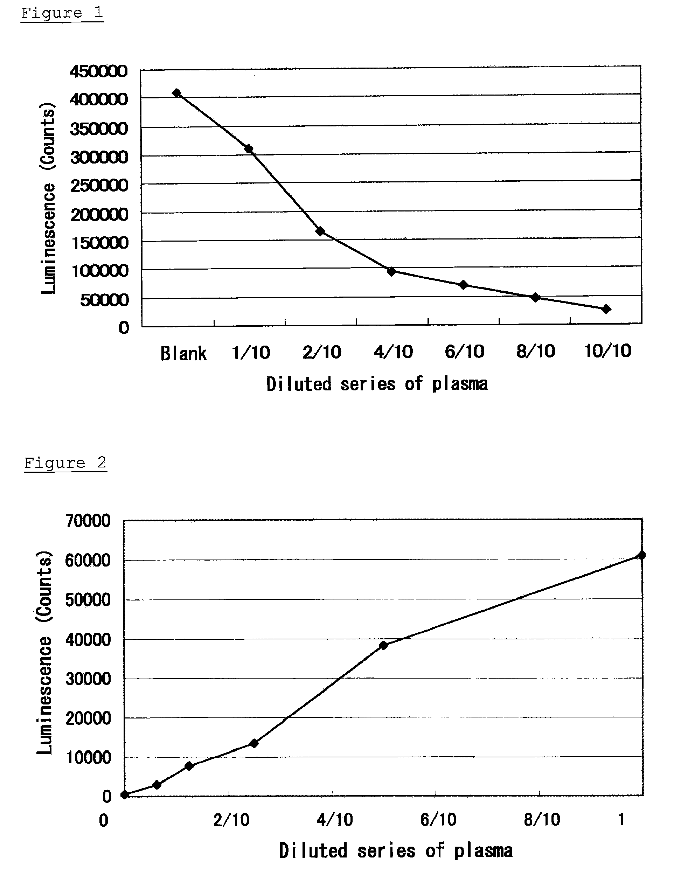

Figure 1 is a graph showing the result of measuring a thrombin activity in plasma by the method of analyzing an enzyme according to the present invention. The horizontal axis indicates a dilution rate in a diluted series of plasma, and the vertical axis indicates an amount of luminescence (unit = counts).
Figure 2 is a graph showing the result of measuring an ADAMTS13 activity in plasma by the method of analyzing an enzyme according to the present invention. The horizontal axis indicates a dilution rate in a diluted series of plasma, and the vertical axis indicates an amount of luminescence (unit = counts).

### BEST MODE FOR CARRYING OUT THE INVENTION

In the method of analyzing an enzyme according to the present invention, an immobilized substrate, i.e., an enzyme substrate which is immobilized on an insoluble carrier and can be cleaved by the enzyme to be analyzed, is used. The method of the present invention includes various embodiments classified by the labeling with a labeling substance, for example,
(1) an embodiment in which the enzyme substrate carried on an insoluble carrier is labeled with a labeling substance (hereinafter sometimes referred to as a non-immunologically analyzing method), and
(2) an embodiment using a labeled partner which is labeled with a labeling substance and specifically binds to a neoantigen which is newly generated by a cleavage of the enzyme substrate with the enzyme (hereinafter sometimes referred to as an immunologically analyzing method).

An enzyme which may be analyzed by the method of the present invention is not particularly limited, so long as it is an enzyme capable of cleaving a substrate at a specific site. As the enzyme, there may be mentioned, for example, an enzyme which may be a subject to be tested in a clinical diagnosis (such as an enzyme in which an increase or decrease level is observed in a human disease), an enzyme not involved in human diseases, or an enzyme derived from animals other than a human.

A test sample to be analyzed is not particularly limited, so long as it is a sample suspected of containing an enzyme to be analyzed. The test sample may be, for example, a biological liquid sample, particularly, blood, serum, plasma, urine, spinal fluid, or the like.

An enzyme substrate used in the present invention is not particularly limited, so long as it is a substance which may be specifically cleaved by an enzyme to be analyzed. The enzyme substrate may be appropriately selected according to an enzyme to be analyzed, and may be prepared by well-known methods. As such well-known methods, there may be mentioned, for example, a method in which a crude extract or a purified substance of a parasite is collected from a body fluid derived from a human or animals, a method in which a crude extract or a purified substance derived from an organism is obtained from an artificially cultured organism, a method in which a nucleic acid or a protein is produced by gene recombination techniques and cell culture techniques, or a method in which a polypeptide or a hapten is synthesized by chemical synthetic techniques.

When a synthetic substance is used as the enzyme substrate, for example, a peptide comprising Gly-Pro-Arg may be used for thrombin. Similarly, a peptide comprising, for example, Gln-Gly-Arg for factor XII, Glu-Lys-Lys for plasmin, Ile-Glu-Gly-Arg for factor X, or Pyr-Gly-Arg for tissue plasminogen activator inhibitor, may be used. Further, for example, a fragment consisting of amino acids 1596-1668 of von Willebrand Factor (vWF) may be used for ADAMTS13 (Koichi Kokame, Masanori Matsumoto, Yoshihiro Fujimura, and Toshiyuki Miyata, VWF73, a region from D1596 to R1668 of von Willebrand factor, provides a minimal substrate for ADAMTS-13. Blood, Jan 2004; 103: 607 - 612).

As a carrier for immobilization used in the present invention, an insoluble carrier having any form may be used, so long as the enzyme substrate can be immobilized thereon. As such a carrier, for example, a plate, a tube, beads, or a tip made of a material (such as polystyrene) capable of physically adsorbing a substrate; a membrane, latex particles, a magnetic carrier, or a glass having an appropriate functional group for the immobilization of a substrate, may be used. Particles are preferable, because such an immobilized substrate can be easily separated from a reaction system, and insoluble magnetic particles are more preferable, because the separation can be carried out using an apparatus.

The particle diameter of the insoluble magnetic particle is generally 0.05 µm to 5 µm, preferably within a range of between 0.3 µm to 3 µm. As a physical adsorption method, there may be mentioned, for example, a method in which a substrate is directly immobilized on insoluble magnetic particles. As a chemical immobilization, there may be mentioned, for example, a method in which a substrate is directly immobilized on magnetic particles by utilizing a functional group (such as an amino, carboxyl, mercapto, hydroxyl, aldehyde, or epoxy group) which exists on the surface of the magnetic particles and can bind to the substrate by a chemical modification of the functional group, or a method in which a substrate is immobilized by introducing a spacer molecule between the magnetic particle and the substrate molecule via a chemical bind. As an example of the spacer molecule, an immobilized substrate may be prepared by chemically immobilizing avidin or streptavidin on insoluble magnetic particles and binding thereto a biotinylated substrate. Further, a method in which an antibody or an antigen is chemically linked to a protein such as albumin, and the resulting conjugate is chemically linked to the particles, may be exemplified. An amount of a substrate generally carried on particles may be appropriately determined in accordance with a surface area, an amount of a functional group, or the like of insoluble carrier particles used, and is generally 1 mg to 500 mg, preferably 10 mg to 100 mg, with respect to 1 g of carrier particles.

A partner substance used in the immunologically analyzing method in the method of the present invention is not particularly limited, so long as it is a substance capable of specifically binding to a neoantigen which is newly generated by a cleavage of an enzyme substrate with an enzyme to be analyzed. As the partner substance, there may be mentioned, for example, an antibody. When a sugar chain is added to a substrate, concanavalin A or lectin which recognizes a specific sugar chain may be used as the partner substrate. The neoantigen may be a neoantigen newly generated on a substrate fragment which is released from the substrate carried on an insoluble carrier, or a neoantigen newly generated on a substrate fragment which remains on an insoluble carrier. The neoantigen newly generated on a substrate fragment which remains on an insoluble carrier is preferable. When an antibody is used as the partner substance, the antibody may be appropriately selected in accordance with an enzyme to be analyzed. An antiserum obtained by immunizing a substrate or a fragment thereof, an affinity-purified polyclonal antibody, or a monoclonal antibody to a human, mouse, rabbit, rat, goat, sheep, or the like may be used. A monoclonal antibody is preferable to avoid a nonspecific binding.

As a labeling substance used in the present invention, various known labeling substances, for example, a fluorescent substance such as europium, a luminescent substance such as luciferin, a coloring substance such as para-nitroaniline (pNA), or an enzyme such as peroxidase, may be used. An enzyme is preferable in view of the stability of a reagent and the sensitivity. As the enzyme, there may be mentioned, for example, peroxidase, alkaline phosphatase, glucose oxidase, β-galactosidase, urease, or luciferase. In particular, a method of measuring an activity using alkaline phosphatase and 1,2-dioxetane as a substrate is preferable for a measurement of a trace component, because a very strong signal is obtained in this reaction system and a high detection sensitivity can be achieved.

In the present invention, an enzyme substrate immobilized on an insoluble carrier (i.e., immobilized substrate) is labeled with a labeling substance in the non-immunologically analyzing method, whereas a partner substance (particularly, an antibody) is labeled with a labeling substance in the immunologically analyzing method. In the case of the labeling of an immobilized substrate, a labeling substance is bound at a region which is different from the cleavage site of an enzyme to be analyzed and is removed from an insoluble carrier by the cleavage with the enzyme (for example, the terminus opposite to the immobilized terminus in the substrate). Methods of preparing a labeled substrate (preferably a substrate labeled with an enzyme) or a labeled partner (preferably a partner substance labeled with an enzyme, more preferably an antibody labeled with an enzyme) are well-known, and any method may be selected. For example, a maleimide group is introduced into an enzyme, and a thiol group is introduced into a substrate or an antibody, and both may be reacted with each other to carry out the enzyme labeling. N-hydroxy-sulfosuccinimide ester or the like may be preferably used as an agent for the introduction of a maleimide group. S-acetyl-mercaptosuccinic acid anhydride or the like may be preferably used as an agent for the introduction of a thiol group. The introduced maleimide group may be reacted with the introduced thiol group at pH 7 or less and 4°C overnight to prepare the enzyme-labeled substrate or the enzyme-labeled antibody.

A method of detecting a change in a signal strength derived from a labeling substance may be appropriately selected in accordance with a type of the labeling substance and the properties of a measuring apparatus, and well-known methods, such as a colorimetric method, a fluorescent method, or a luminescent method, may be utilized. For example, when peroxidase is used as a labeling substance, the signal may be detected by a chemiluminescent method using luminol as a substrate, which is particularly preferable for a measurement of a trace component (for example, Koichiro Tanaka and Eiji Ishikawa, Comparison of fluorescent method and luminescent method in enzyme immunoassay using horseradish peroxidase as label, Rinsho-kensa-kiki-shiyaku, 11: 567-569, 1988). When alkaline phosphatase is used as a labeling substance, it is preferable to use known substrates, such as AMPPD [3-(2'-spiroadamantane)-4-methoxy-4-(3"phosphoroxy)phenyl-1,2-dioxetane] or 1,2-dioxetane (CDP-Star; Tropics), because the signal can be detected with a high sensitivity.

Hereinafter the method of analyzing an enzyme according to the present invention will be further illustrated by showing the concrete measuring procedures of various embodiments.
An embodiment of the method of analyzing an enzyme according to the present invention, the non-immunologically analyzing method, may comprise, but is by no means limited to, the following steps. For example, an immobilized substrate, in which a terminus of a substrate specific for an enzyme of interest is labeled and the substrate is immobilized on an insoluble carrier (such as insoluble magnetic particles) via the other terminus thereof, is reacted with a sample containing the enzyme for a predetermined period of time. The immobilized substrate is washed to separate a labeled substrate fragment released from the immobilized substrate by the enzymatic cleavage and the sample, from the immobilized substrate. An enzyme activity contained in the sample can be analyzed by measuring an amount of the labeling substance of the substrate which is not cleaved and remains on the insoluble magnetic particles.

The non-immunologically analyzing method of the present invention comprises, for example, the steps of:
(A1) bringing (a) a sample suspected of containing an enzyme to be analyzed, into contact with (b) a labeled immobilized substrate, i.e., an enzyme substrate which is immobilized on an insoluble carrier and can be cleaved by the enzyme and the enzyme substrate is labeled with a labeling substance at a side of a substrate fragment released from the insoluble carrier by a cleavage of the enzyme, in a liquid,
(A2) separating the insoluble carrier from the liquid, and
(A3) analyzing a substrate which is not cleaved and remains on the insoluble carrier and/or a fragment of the substrate which is released from the insoluble carrier into the liquid.

The labeled immobilized substrate is an enzyme substrate which is linked to an insoluble carrier and labeled with a labeling substance. An enzyme substrate portion in the labeled immobilized substrate contains a specific cleavage site recognized by an enzyme to be analyzed. Further, a site which is different from the cleavage site and is removed from an insoluble carrier by the cleavage with the enzyme (for example, the terminus opposite to the immobilized terminus) in the enzyme substrate portion is labeled with a labeling substance. In the step (A1), when a sample containing an enzyme to be analyzed is brought into contact with the labeled immobilized substrate, the enzyme cleaves the substrate at the specific cleavage site in the labeled immobilized substrate. As a result, a substrate fragment with the label is released from the insoluble carrier, and an immobilized substrate which is not cleaved with the enzyme remains on the insoluble carrier. A contact time in this step is not particularly limited, so long as the reaction of the immobilized substrate with the enzyme suspected of being contained in a sample can be carried out. The contact time may be appropriately determined in accordance with reaction conditions, such as amounts of reagents and a sample, a temperature, or a pH, and is generally 3 minutes to 24 hours, preferably 15 minutes to 1 hour.

In the step (A2), the insoluble carrier after the execution of the step (A1) is separated from the other fraction, i.e., a liquid fraction containing the released substrate fragment and the sample. The separation may be carried out by a conventional method. When magnetic particles are used as the insoluble carrier, the separation can be carried out by, for example, the use of a magnet or centrifugation. When latex particles are used as the insoluble carrier, the separation can be carried out, for example, by centrifugation or filtration. When a labeling substance contained in the insoluble carrier is analyzed in the subsequent step (A3), it is preferable to wash the insoluble carrier after the separation to fully remove therefrom the released substrate fragment.

In the step (A3), either the substrate fragment remaining on the insoluble carrier or the substrate fragment released from the insoluble carrier into the liquid, or both thereof can be analyzed.
When the substrate fragment released from the insoluble carrier into the liquid is analyzed, an amount of the released substrate fragment may be determined by measuring an amount of the labeling substance contained in the released substrate fragment. Further, an amount or activity of an enzyme to be analyzed contained in a sample may be determined, for example, by preparing a calibration curve. When an enzyme to be analyzed is contained in a sample, an amount of the released substrate fragment is increased dependent on an amount or activity of the enzyme.
When the substrate fragment which is not cleaved and remains on the insoluble carrier is analyzed, an amount of the remaining labeled substrate (i.e., immobilized labeled substrate) may be determined by measuring an amount of the remaining labeling substance. Further, an amount or activity of an enzyme to be analyzed contained in a sample may be determined, for example, by preparing a calibration curve. When an enzyme to be analyzed is contained in a sample, an amount of the immobilized labeled substrate is decreased dependent on an amount or activity of the enzyme.

An embodiment of the method of analyzing an enzyme according to the present invention, the immunologically analyzing method, may comprise, but is by no means limited to, the following steps. For example, an immobilized substrate is reacted with a sample for a predetermined period of time. A labeled antibody which specifically binds to a cleaved fragment is added thereto, and a reaction is carried out for a predetermined period of time. The immobilized substrate is washed. An enzyme activity contained in the sample can be analyzed by measuring an amount of the labeled antibody bound to the immobilized substrate.

In the immunologically analyzing method of the present invention, a labeled partner which is labeled with a labeling substance and specifically binds to a neoantigen which is newly generated by cleaving the enzyme substrate with an enzyme to be analyzed is used to carry out the analyzing step. Hereinafter the immunologically analyzing method of the present invention will be illustrated, mainly on the basis of the case in which the neoantigen is generated at the side of a substrate fragment remaining on an insoluble carrier, but it is possible for those skilled in the art to carry out the present invention, with appropriate modifications if necessary, even if the neoantigen is generated at the side of a released substrate fragment. The labeled partner may be added to the reaction system at any step, for example, the contacting step, the separating step, or the analyzing step.

For example, when the labeled partner is added in the contacting step, the addition may be carried out, before the contact of a sample with the immobilized substrate (i.e., the labeled partner is brought into contact with one of a sample or the immobilized substrate, followed by the other), simultaneously with the contact (i.e., a sample, the immobilized substrate, and the labeled partner are brought into contact with each other at the same time), or after the contact (i.e., a sample is brought into contact with the labeled substrate, and then, the mixture is further brought into contact with the labeled partner). Because the labeled partner specifically binds to a neoantigen which is newly generated by cleaving an enzyme substrate with an enzyme to be analyzed, the labeled partner does not bind to the immobilized substrate before the enzymatic cleavage, and the enzymatic reaction in the contacting step proceeds independently from the presence or absence of the labeled partner. Where the labeled partner is added in the contacting step, a sample contains an enzyme to be analyzed and the immobilized substrate is cleaved with the enzyme, and, as a result, a complex (preferably immunocomplex) of the substrate fragment (immobilized substrate fragment or released substrate fragment) and the labeled partner (preferably labeled antibody) is formed.

When the labeled partner is added in the separating step, the addition may be carried out before or after the separation. For example, where the labeled partner is added after the separation and the labeled partner recognizes a neoantigen which is generated at the side of a substrate fragment remaining on an insoluble carrier, the labeled partner may be added to an insoluble carrier obtained by the separation, and then, an unreacted labeled partner may be removed by washing the insoluble carrier.

When the labeled partner is added in the analyzing step, for example, the labeled partner may be added to an insoluble carrier obtained by the separating step, and then, the insoluble carrier may be washed to remove an unreacted labeled partner, and the labeled partner may be analyzed.

It is preferable to add the labeled partner in the contacting step, or before the separation in the separating step, because a procedure for separating the reacted labeled partner from the unreacted labeled partner may be omitted.

In the analyzing step of the immunologically analyzing method, for example, when the labeled partner recognizes a neoantigen which is generated at the side of a substrate fragment remaining on an insoluble carrier, an amount or activity of an enzyme to be analyzed contained in a sample may be determined by analyzing the labeled partner specifically bound to a substrate fragment remaining on an insoluble carrier. That is, an amount of the immobilized substrate fragment may be determined by measuring an amount of a labeled substance of the labeled partner specifically bound to a substrate fragment remaining on an insoluble carrier. Further, an amount or activity of an enzyme to be analyzed contained in a sample may be determined, for example, by preparing a calibration curve. When an enzyme to be analyzed is contained in a sample, an amount of the immobilized substrate fragment is increased dependent on an amount or activity of the enzyme, and therefore, an amount of the labeled partner specifically bound to the substrate fragment is also increased.

The kit of the present invention for analyzing an enzyme comprises at least the immobilized substrate, i.e., an enzyme substrate which is immobilized on an insoluble carrier and can be cleaved by the enzyme. The kit of the present invention for analyzing an enzyme includes a kit which may be used in the non-immunologically analyzing method of the present invention (hereinafter referred to as a kit for an non-immunological analysis) and a kit which may be used in the immunologically analyzing method of the present invention (hereinafter referred to as a kit for an immunological analysis).

The kit for a non-immunological analysis of the present invention comprises at least the labeled immobilized substrate, i.e., an enzyme substrate which is immobilized on an insoluble carrie and can be cleaved by an enzyme to be analyzed, wherein the enzyme substrate is labeled with a labeling substance at the side of a substrate fragment released from the insoluble carrier by a cleavage with the enzyme.
The kit for an immunological analysis of the present invention comprises at least the immobilized substrate, i.e., an enzyme substrate which is immobilized an insoluble carrier and can be cleaved by the enzyme, and the labeled partner which is labeled with a labeling substance and specifically binds to a neoantigen which is newly generated by cleaving the enzyme substrate with the enzyme.

The kit of the present invention for analyzing an enzyme may further comprise, for example, an enzyme substrate, a washing solution, a solution for diluting a sample, and/or an amplifier, in accordance with a labeling substrate and a detection system. For example, a first reagent-component may contain the immobilized substrate, and a second reagent-component may contain a substance capable of expressing a signal from a labeling substance. More particularly, for example, as the kit for a non-immunological analysis, a reagent-component system consisting of a first reagent-component: a solution of an immobilized substrate (labeled with alkaline phosphatase), and a second reagent-component: a substrate for chemiluminescence (such as AMPPD) may be exemplified and, as the kit for an immunological analysis, a reagent-component system consisting of
a first reagent-component: a solution of an immobilized substrate,
a second reagent-component: a labeled antibody specifically recognizing a cleaved fragment (labeled with alkaline phosphatase), and
a third reagent-component: a substrate for chemiluminescence (such as AMPPD)
may be exemplified. These constitutions may be appropriately changed or modified in accordance with a labeling substance and a detection system.

Examples of measurements using these kits for analysis will be concretely illustrated.
A sample solution suspected of containing an enzyme to be measured is reacted in a liquid, together with a first reagent-component consisting of insoluble magnetic particles carrying a substrate which contains a cleavage site of the enzyme and is labeled with a labeling enzyme, in a reaction vessel. Alternatively, a sample solution suspected of containing an enzyme to be measured, the above first reagent-component, and a second reagent-component consisting of an antibody which recognizes a cleavage site in a substrate carried on insoluble magnetic particles, are reacted with each other in a liquid medium in a reaction vessel. The first and second reagent-components may be added in a liquid medium into the reaction vessel, simultaneously or in a stepwise fashion, and the order of addition is not limited.

After the addition, the mixture should be fully mixed. The resultant mixture may be allowed to stand without mixing, to carry out a reaction. The reaction is carried out, generally at pH 5 to 10, preferably at pH 6 to 7, as conventional immunochemical reactions. The reaction may be carried out, generally at 2 to 50°C, preferably from room temperature to a temperature within a range of 37 to 45°C. The reaction time may be selected from immediately after the reaction to a whole day and night, but is generally 3 to 60 minutes in view of sensitivity and operability.

A buffer solution is usually used to maintain a desired pH. As such a buffer solution, for example, MES (2-Morpholinoethansulphonic Acid), phosphate, or the like may be used, and almost all buffer solutions commonly used in a weak acidic to neutral region may be used. Salts such as sodium chloride and proteins such as bovine serum albumin are often used, to avoid nonspecific reactions.

A content of the insoluble magnetic particles is generally 0.0001 to 1% by weight, preferably 0.001 to 0.1% by weight, with respect to a reaction mixture. A content of the enzyme-labeled antibody is generally 0.00001 to 0.1 mAbs, preferably 0.0001 to 0.01 mAbs, with respect to a reaction mixture.

### EXAMPLES

The present invention now will be further illustrated by, but is by no means limited to, the following Examples.

### Example 1: Measurement of thrombin activity

### (1) Labeling of substrate

A synthetic peptide substrate of which the C-terminus was labeled with biotin (N-Gly-Pro-Arg-biotin) was synthesized with an automatic peptide synthesizer, in accordance with an Fmoc chemistry using 1,2-diaminoethanetrityl resin and N-α-tert-butoxycarbonylglycine as an N-terminal amino acid residue of a peptide. After the C-terminal amino acid of the peptide was removed from the resin, 5-(N-succinimidyloxycarbonyl)pentyl D-biotinamide was used to label the peptide with biotin via 1,2-diaminoethane. All protective groups were removed from the side chains in the biotin-labeled peptide by reacting the peptide with trifluoroacetic acid. A commercially available alkaline phosphatase labeling kit (Dojin, code: LK12) was used to label the N-terminal amino group of N-Gly-Pro-Arg-biotin with alkaline phosphatase.

### (2) Binding substrate to magnetic particles

A 1% suspension of streptavidin-labeled magnetic particles (Dynal Biotech, particle diameter = 2.8 µm) was reacted with the labeled substrate (0.5 mg/mL) prepared in the above section (1) in a 50 mmol/L MES buffer (pH 6) for 1 hour. The particles were washed with the MES buffer and blocked with a 0.1 mol/L Tris buffer (pH 8) containing 0.3% bovine albumin, to prepare magnetic particles carrying the labeled substrate (labeled-substrate-bound magnetic particles).

### (3) Measurement of thrombin in plasma

A compact automated immunoassay analyzer (PATHFAST; Mitsubishi-Kagaku Iatron) was used to carry out the following measurement. A diluted series (60 µL) of plasma containing a predetermined amount of thrombin (0.1 IU/mL) was reacted with a suspension (100 µL) of the labeled-substrate-bound magnetic particles at 37°C for 5 minutes. After the magnetic particles were washed with 0.1 mol/L Tris buffer (pH 8)/0.15 mol/L NaCL/0.1% Triton X-100, a solution (100 µL) of CDP-Star (Tropics) as a chemiluminescent substrate solution was mixed with the magnetic particles. A reaction was carried out at 37°C for 4 minutes, and an amount of luminescence was counted. As a blank, these procedures were repeated except that a physiological saline was used instead of the plasma. The result is shown in Figure 1.

As apparent from the graph shown in Figure 1, when the concentration of thrombin was high, the immobilized substrate was cleaved, a substrate fragment labeled with the enzyme was released from the particles, an amount of the substrate remaining on the particles was decreased, and as a result, the count of luminescence was decreased in dependence on the concentration. A concentration or activity of thrombin contained in a sample can be quantitatively determined by preparing a calibration curve using a standard reagent or plasma containing a predetermined concentration or activity of thrombin.

### Example 2: Measurement of ADAMTS13 activity

### (1) Preparation of substrate

As a substrate for ADAMTS13 [von Willebrand Factor (vWF) cleaving protease], a partial fragment of the vWF (a fragment consisting of amino acids 1596-1668; hereinafter referred to as vWF73) was prepared in accordance with the method described in Koichi Kokame, Masanori Matsumoto, Yoshihiro Fujimura, and Toshiyuki Miyata, VWF73, a region from D1596 to R1668 of von Willebrand factor, provides a minimal substrate for ADAMTS-13. Blood, Jan 2004; 103: 607 - 612.

### (2) Bonding substrate to magnetic particles

An 1% solution of magnetic latex particles (manufactured by JSR) having a particle diameter of 2.4 µm was reacted with the vWF73 (500 µg) prepared in the above section (1) in a 50 mmol/L MES buffer (pH 6) at room temperature for 1 hour. The particles were washed with the MES buffer and blocked with a 0.1 mol/L Tris buffer (pH 8) containing 0.3% bovine albumin, to prepare magnetic particles carrying the substrate (substrate-bound magnetic particles).

### (3) Labeling of vWF monoclonal antibody with alkaline phosphatase

A monoclonal antibody specifically recognizing the cleavage site of ADAMTS13 in the vWF73 was prepared in accordance with the method described in Cruz MA, Whitelock J, Dong JF. Evaluation of ADAMTS13 activity in plasma using recombinant von Willebrand Factor A2 domain polypeptide as substrate. Thromb Haemost. 2003; 90(6):1204-9. A protein G column was used to purify an IgG from an antiserum. The IgG was digested with 4% pepsin in a 0.1 mol/L sodium acetate solution (pH 3.9) at 37°C overnight, to prepare an F(ab')₂. A 2-mercaptoethylamine solution was further added thereto, and a reaction was carried out at 37°C for 90 minutes. An Ultrogel AcA44 column was used to purify an Fab'. Alkaline phosphatase was dialyzed in a 50 mmol/L sodium borate buffer (pH 7.6) containing 1 mmol/L magnesium chloride and 0.1 mmol/L zinc chloride. To this dialyzed solution, N-(6-maleimidocaproyloxy)succinimide was added, and the whole was incubated at 30°C for 30 minutes. The resultant solution was concentrated by a gel filtration through a Sephadex G-25 column, to prepare a maleimide-introduced alkaline phosphatase. This maleimide-introduced alkaline phosphatase (10 nmol) in a 0.1 mol/L Tris buffer (pH 7) containing 1 mmol/L magnesium chloride and 0.1 mmol/L zinc chloride was mixed with the Fab' (50 nmol) in a 0.1 mol/L sodium phosphate buffer (pH 6) containing 5 mmol/L EDTA, and this mixture was incubated at 4°C for 20 hours. A 10 mmol/L 2-mercaptoethylamine solution was further added thereto, and a gel filtration was carried out through an Ultrogel AcA44 column with a 0.1 mol/L Tris buffer (pH 6.8) containing 0.1 mmol/L sodium chloride, 1 mmol/L magnesium chloride, and 0.1 mmol/L zinc chloride, to obtain an enzyme-labeled Fab'.

### (4) Measurement of ADAMTS13 activity

A compact automated immunoassay analyzer (PATHFAST; Mitsubishi-Kagaku Iatron) was used to carry out the following measurement. A diluted series (60 µL) of normal human plasma was reacted with a suspension (100 µL) of the substrate-bound magnetic particles prepared in the above section (2) at 37°C for 5 minutes. The anti-vWF monoclonal antibody labeled with alkaline phosphatase (1 mAbs.), prepared in the above section (3), was further added thereto, and a reaction was carried out at 37°C for 4 minutes. After the magnetic particles were washed with 0.1 mol/L Tris buffer (pH 8)/0.15 mol/L NaCL/0.1% Triton X-100, a solution (100 µL) of CDP-Star (Tropics) as a chemiluminescent substrate solution was mixed with the magnetic particles. A reaction was carried out at 37°C for 2 minutes, and an amount of luminescence was counted. As a blank, these procedures were repeated except that a physiological saline was used instead of the plasma. The result is shown in Figure 2.

### INDUSTRIAL APPLICABILITY

The method of analyzing an enzyme according to the present invention can be applied to clinical diagnostic tests.
Although the present invention has been described with reference to specific embodiments, various changes and modifications obvious to those skilled in the art are possible without departing from the scope of the appended claims.

### FREE TEXT IN SEQUENCE LISTING

Features of "Artificial Sequence" are described in the numeric identifier <223> in the Sequence Listing. The amino acid sequence of SEQ ID NO: 1 is a synthetic substance.

## Claims

1. A method of analyzing an enzyme, **characterized by** comprising the steps of:
(1) bringing a sample suspected of containing an enzyme to be analyzed into contact with an enzyme substrate which is immobilized on an insoluble carrier and can be cleaved by the enzyme, in a liquid,
(2) separating the insoluble carrier from the liquid, and
(3) analyzing a substrate or a fragment thereof which remains on the insoluble carrier and/or a fragment of the substrate which is released from the insoluble carrier into the liquid.

2. The method according to claim 1, wherein the enzyme substrate is labeled with a labeling substance at a side of a substrate fragment released from the insoluble carrier by a cleavage with the enzyme to be analyzed.

3. The method according to claim 1, wherein the step (3) is carried out by using a labeled partner which is labeled with a labeling substance and specifically binds to a neoantigen which is newly generated by a cleavage of the enzyme substrate with the enzyme to be analyzed.

4. The method according to claim 3, wherein the labeling partner is a labeling antibody.

5. The method according to any one of claims 2 to 4, wherein the labeling substance is a fluorescent substance, a luminescent substance, a coloring substance, or an enzyme.

6. The method according to any one of claims 1 to 5, wherein the insoluble carrier is a particle.

7. The method according to claim 6, wherein the particle is a latex particle or a magnetic particle.

8. A kit for analyzing an enzyme, **characterized by** comprising
an enzyme substrate which is immobilized on an insoluble carrier and can be cleaved by the enzyme, wherein the enzyme substrate is labeled with a labeling substance at a side of a substrate fragment released from the insoluble carrier by a cleavage with the enzyme.

9. A kit for analyzing an enzyme, **characterized by** comprising
an enzyme substrate which is immobilized on an insoluble carrier and can be cleaved by the enzyme, and
a labeled partner which is labeled with a labeling substance and specifically binds to a neoantigen which is newly generated by cleaving the enzyme substrate with the enzyme.
